# EUROPEAN PATENT APPLICATION

(11) **EP 2 989 974 A1**
(43) Date of publication of application: **02.03.2016**
(21) Application number: 15182901.7
(22) Date of filing: 28.08.2015
(51) Int. Cl.: A61B 5/00, A41D 1/00, A41D 13/12, A61B 5/0205, A61B 5/01, A61B 5/024, A61B 5/02, A61B 5/11

(54) **A MODULE INSTALLABLE WITH A GARMENT, THE CORRESPONDING GARMENT AND A METHOD OF MANUFACTURING THE MODULE OR THE GARMENT**

(30) Priority: 28.08.2014 FI 20145747
(71) Applicant: Clothing Plus MBU Oy, 38700 Kankaanpää (FI)
(72) Inventor: REHO, Akseli, 38700 Kankaanpää (FI); JAAKKOLA, Heikki, 38700 Kankaanpää (FI); ILKKO, Aino-Maria, 38700 KANKAANPÄÄ (FI)
(74) Representative: Berggren Oy Ab

(57) **Abstract**

Electric components (102) to be installed into or onto a garment (101) are provided in/on a module (100). The module (100) comprises an installation zone (103) via which said module (100) with said components (102) is configured to be installed, such as sewed, quilted, glued, welded or laminated, with the garment in a fixed manner. The installation zone (103) is arranged so that the module (100) is also configured to be detached (108) from the garment (100) via said installation zone (103, 108) by breaking, such as cutting or tearing the structure of said installation zone (103).

## Description

### TECHNICAL FIELD OF THE INVENTION

The invention relates to a module installable with a garment and the garment comprising the module installed with the garment. In addition the invention relates to a method for manufacturing the module or garment.

### BACKGROUND OF THE INVENTION

Garments with electric components, such as measuring electronics, are commonly known from prior art, where the electric components are typically integrated into or onto the structure of the garment at a manufacturing stage of the garment. The field of this technology is commonly known as a wearable technology or smart clothing. Very often the garment manufacturer needs to manufacture and install one electric component to a certain location, another electric component to another location and provide suitable power and signal lines between the components. Thus the garment manufacturer needs to have also knowledge also about the field of electronics. Other possibility is that the manufacturer sends a half-finished garment to an electronic component manufacturer, who manufactures the components and installs them into or onto the half-finished garment and again sends the half-finished garment with the installed electronic components to the garment manufacturer for the finishing stage. One problem is that there are only few professionals who can manage the wearable technology or installation of the electronic components to the garments or smart clothing.

Thus there are some disadvantages relating to the known prior art, such as logistical disadvantages when the manufacturer of different technical fields need to send half-finished products to each other. In addition designing of the electronic components raises often numbers of special issues which are not always very clear for the manufacturers of the garments. Furthermore there is a disadvantage relating to recycling of the garments after their lifetime, because they includes electronic components with materials, like metals and especially heavy metals used in electronic components and batteries, which should be recycled separately from the garment material, like textile or fabric. For example the components integrated into the structure of the garment might be very difficult to find and remove.

### SUMMARY OF THE INVENTION

An object of the invention is to alleviate and eliminate the problems relating to the known prior art. Especially the object of the invention is to provide a solution how to simplify the logistic between the electronic component manufacturer and the garment manufacturer. In addition the object is to fasten and facilitating the manufacturing process of the garments with electronic components so that there is no need for garment manufacturer to have any deep know-how about the technical field of field of electronics.

The object of the invention can be achieved by the features of independent claims.

The invention relates to a module installable with a garment according to claim 1. In addition the invention relates to the garment comprising the module according to claim 11, as well as to a manufacturing method of the module or garment according to claim 18.

According to an embodiment of the invention electric components to be installed into or onto a garment are provided in/on a module. The module is provided with an installation zone via which the module is configured to be installed with the garment in a fixed manner. In addition the installation zone is arranged so that the module can also be detached from the garment via said installation zone by breaking, such as cutting or tearing the structure of said installation zone. The installation zone may comprise weaker portion or line, where the material strength is weaker than in the vicinity of said portion or line of the installation zone so that the installation zone is thus configured to be broken essentially along said weaker portion or line when applied a physical force, such as tearing. The weaker portion or line may be achieved by a material choice or for example a perforation may be used.

Advantageously the installation zone is arranged so that the module with the electric component can also be detached from the garment via said installation zone advantageously by breaking the structure of said installation zone physically so that at least portion of said structure of said installation zone is remained in said garment after the breaking, but all the electric components included in said module are removed. Alternatively the installation zone may be arranged so that the whole structure of said installation zone is removed when breaking the structure of said installation zone.

The module can be installed to the garment via the installation zone e.g. by sewing, quilting, gluing, welding or laminating, for example, or other method known in the field of textile industry, depending among others of the material of the module as well as the garment. The substrate material of the module (as well as the garment) may comprise e.g. textile, fabric, plastic, rubber, fibre based material such as fiberglass or cardboard, elastic band, leather, silicone and/or metal.

The electric component to be used in/on the module is for example a sensor for detecting biosignals, such as heart rate or respiration rate, but the sensor may also be used for determining other parameters of environment or a user of a garment, such as posture of the user, temperature, humidity, conductivity or acceleration, as an example. The electric component may also comprise an electric energy storage, battery, capacitor, solar cell, light emitting device, LED, speaker, data processing device (e.g. processor), memory, data transmitting device like antenna with suitable modules, such as Bluetooth module or the like, heating or cooling element, an induction loop for transferring power or data, camera, user interface device such as a button, touch sensor or vibrator, and/or a positioning device.

The module may be an autonomous unit having all electric components for a certain purpose, such as for measuring, data transmitting or signalling. The module may alternatively comprise an interface to be electrically coupled with the garment for example for data or power transmission. Also the garment may comprise a corresponding interface. The interface may be implemented e.g. by a snap fastener, plug and socket, connector, or at least one electrically conductive area, such as conductive areas at the module and the garment at the corresponding position so that the corresponding conductive areas of the module and the garment are arranged to be faced with each other and thereby establishing an electric coupling.

According to an embodiment the module is arranged so that the electric component can be coupled electrically and/or physically via the installation zone(s) with at least one another module. This offers possibility to functionally, such as electrically, couple numbers of modules to form a larger assembly, where for example one module comprises power source, second module comprises measuring electronics such as sensors and third module data transmission device, like wireless transmitter or the like, whereupon the combination of the modules forms a measuring assembly. According to an embodiment the module may comprise conductor wires for transmitting data and/or power, whereupon the module may be used to couple at least one another module having electric components to an additional module of the garment.

According to an embodiment the module may be a structure portion of the garment, such as a portion of a sleeve, back or front portion of a shirt or other garment, leg, pocket, brand label, elastic portion of the garment, or a collar portion of the garment. In addition the module may also be used as a connecting structure between at least two different portions of the garment.

The invention relates also to a garment comprising the module installed with the garment, the module being any module according to any embodiment describer in this document. The garment is for example a shirt, jacket, trousers, swimming suit, band, underwear, shoe, glove or hat or any other garment. According to an embodiment the module is an auxiliary portion installed with the garment or the module (installation) may also be a structure portion of the garment, such as a portion of sleeve, front or back portion of a shirt, leg, pocket, brand label, elastic portion of the garment or a collar portion, for example. The module may also be used, when installed to the garment, as a connecting structure between at least two different portions of the garment, such as connecting or coupling a sleeve element with the base portion of a shirt. It is to be noted that the module may be installed in number ways with the garment, as described in this document. In addition the garment may comprise layers configured to encapsulate the module at least partly between the layers so that at least one layer is arranged as an installation zone.

The present invention offers advantages over the know prior art, such as simplifies logistics between an electronic component manufacturer and garment manufacturer as well as fastens and facilitates the manufacturing process of the garments having electronic components so that there is no need for the garment manufacturer to have any deep know-how about the technical field of field of electronics. In addition the invention makes it easier to recycle the garments after their lifetime, because any electronic components are very easy to find and detach by breaking the material of the installation zone of the module having the electronic components.

### BRIEF DESCRIPTION OF THE DRAWINGS

Next the invention will be described in greater detail with reference to exemplary embodiments in accordance with the accompanying drawings, in which:
- Figures 1-12: illustrate principles of different exemplary modules and installations to garments according to an advantageous embodiment of the invention, and
- Figure 13: illustrate an exemplary garment having the module according to an advantageous embodiment of the invention.

### DETAILED DESCRIPTION

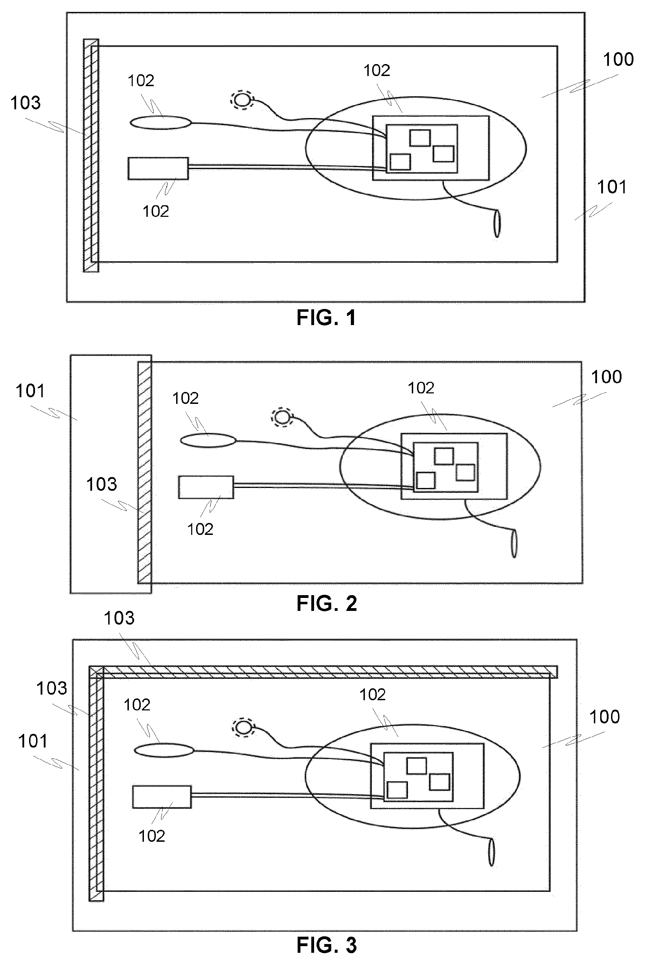

Figures 1-12 illustrate principles of different exemplary modules 100 and installations to garments 101 according to an advantageous embodiment of the invention. The modules 100 comprises electric components 102, and are provided with an installation zone 103 via which the module 100 is configured to be installed with the garment 101 in a fixed manner, such as e.g. by sewing, quilting, gluing, welding or laminating.

The installation zone 103 may be provided on an edge or end portion or side portion of the module, at a rim portion around the module, or at a cover surface of the module 100, as is described in Figures 1 and 2, where one borderline of the module is as said installation zone 103. The module 100 may be covered at least in its one surface by the garment material, as is described in Figure 1, or the module may be as an external module locating at least partially outside the actual garment material, as is described in Figure 2.

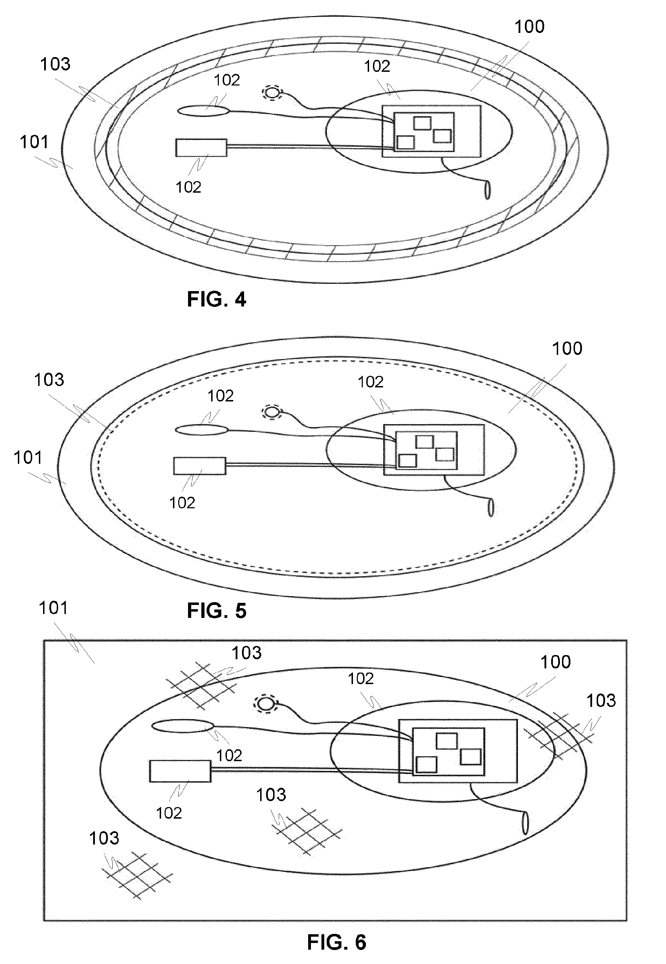

The installation zone 103 may be provided also on at least two sides portion of the of the module 100, as is described in Figures 3, whereupon the fastening of the module into the garment is typically stronger than in the embodiment described in Figured 1 and 2. In addition, according to an embodiment, the installation zone 103 may be provided at a rim portion around the module 100, as is described in Figures 4 and 5. In Figure 5 the installation zone 103 is implemented by sewing or quilting, and in Figure 4 by gluing, welding or laminating, for example.

Figure 6 illustrates an embodiment, wherein the module 100 comprises numbers of installation zones 103 via which it is installed into the garment 101.

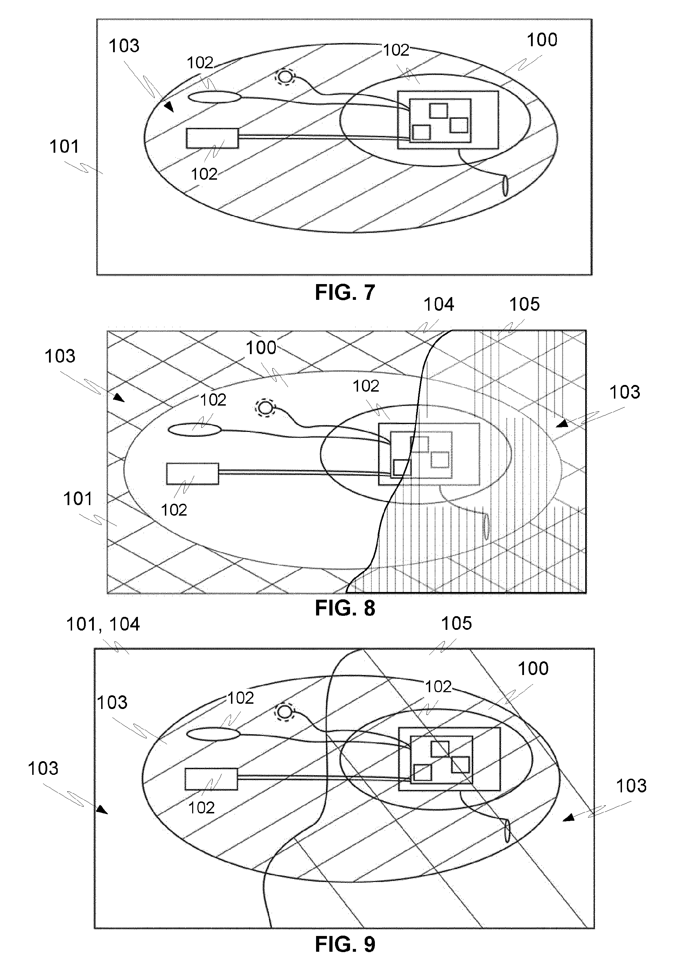

Figure 7 illustrates an example of the module 100, where at least one surface or portion of the surface of the module is as said installation zone 103. Figure 8 illustrates an example of the module 100, where the module 100 is encapsulated between two layers 104, 105, such as textile or fabric layers. The layers 104, 105 are configured to form a cavity for the module, and at least one layer 104, 105 or area surrounding area of the module 100 limited by said layers 104, 105 is arranged as said installation zone 103.

Figure 9 illustrates an example of the module 100, where the module 100 is arranged between two layers 104, 105, such as textile or fabric layers, or where the first layer 104 may also be a layer of garment 101. The module 100 is installed into the garment 101 e.g. by laminating or gluing via the first layer 104 for example via the bottom portion of the module, and the second layer 105 is installed, such as laminated or glued, above the module 100. Thus the installation zone 103 is at least portion or whole are of the module 100 and the surrounding area 104, 105 of the module 100 limited by said layers 104, 105.

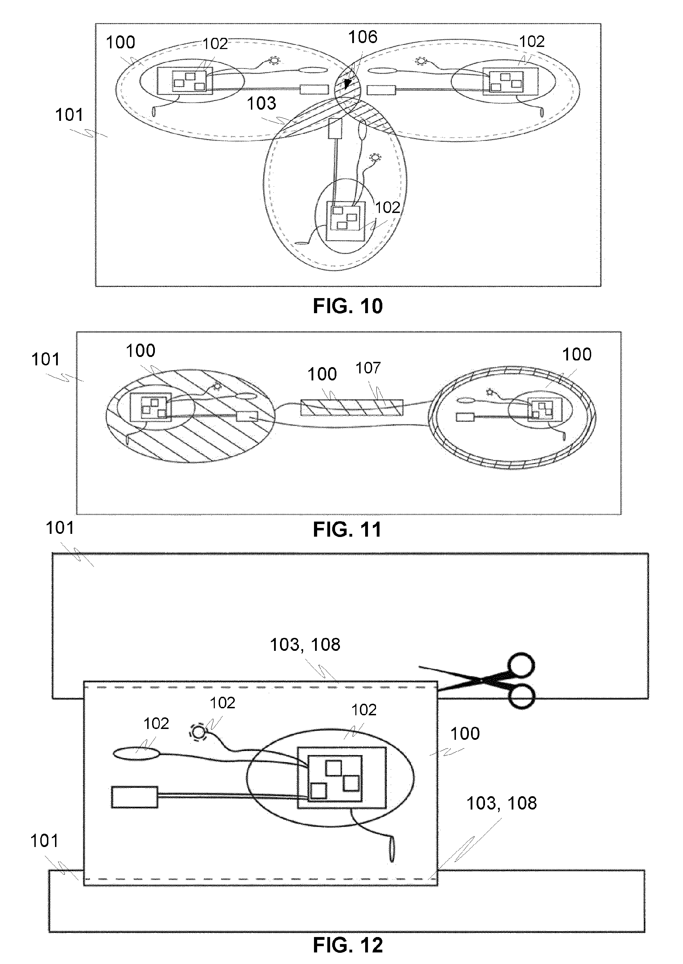

The installation zones 103 are advantageously arranged so that the module 100 can also be detached 108 from the garment 101 via said installation zones 103 by breaking, such as cutting or tearing, the structure of said installation zone 103, as can be seen also in Figure 12.

The module 100 may comprise also interface devices 106, via which it can be coupled electrically and/or physically via the installation zone(s) with at least one another module, as is described in Figure 10. The module may also comprise conductor wires 107 for transmitting data and/or power, whereupon the module 100 may be coupled with at least one another module 100, such as module having electric components 102, or with an electric or data interface of the garment or with an additional module of the garment, as is described in Figure 11.

According to an embodiment the module 100 may be a structure portion of the garment, and/or the module 100 may also be used as a connecting structure between at least two different portions of the garment, as can be seen also in Figure 12.

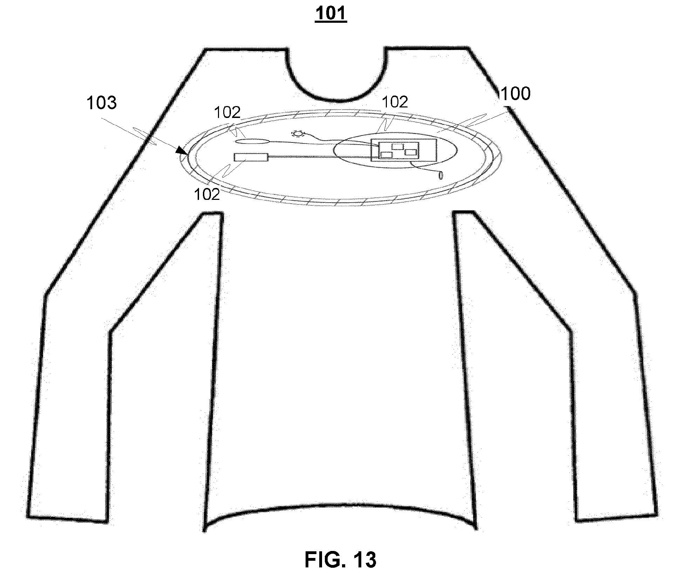

Figure 13 illustrate an exemplary garment 101 having the module 100, where the module is installed into the garment via the installation zone 103 in a fixed manner, such as sewed, quilted, glued, welded or laminated. In addition the installation zone 103 is arranged so that the module 101 can be detached from the garment 101 via the installation zone 103 by breaking, such as cutting or tearing the structure of the installation zone 103.

The invention has been explained above with reference to the aforementioned embodiments, and several advantages of the invention have been demonstrated. It is clear that the invention is not only restricted to these embodiments, but comprises all possible embodiments within the spirit and scope of the inventive thought and the following patent claims. For example it is to be noted that at least one of the electric component of the module may be installed in/on the module in a removable and re-installable manner.

## Claims

1. A module (100) installable with a garment (101),
wherein the module comprises:
- an electric component (102) and,
- an installation zone (103) via which said module is configured to be installed with the garment in a fixed manner,
**characterized in that**
- said electric component (102) comprises a sensor for detecting biosignals of a user during wearing said garment, and
- said installation zone is arranged so that the module with the electric component is also configured to be detached (108) from the garment via said installation zone (103, 108) by breaking the structure of said installation zone (103) physically so that at least portion of said structure of said installation zone is remained in said garment after the breaking, but all the electric components are removed.

2. A module of claim 1, wherein a substrate of the module comprises textile, fabric, plastic, rubber, fibre based material, elastic band, leather, silicone or metal.

3. A module of any of previous claims, wherein the installation zone (103) is provided on an edge or side portion of the module, at a rim portion around the module, or at a cover surface of the module.

4. A module of any of previous claims, wherein the module is encapsulated between two surface layers (104, 105), and wherein the at least one surface layer is arranged as said installation zone.

5. A module of any of previous claims, wherein the module is configured to be a structure portion of the garment, such as a portion of a sleeve, back or front portion of a shirt, leg, pocket, brand label, elastic portion of the garment, a collar portion.

6. A module of any of previous claims, wherein the electric component is a sensor (102) for detecting heart rate, respiration rate, posture of the user, temperature, humidity, conductivity, acceleration, or wherein the electric component additionally comprises an electric energy storage, battery, capacitor, solar cell, light emitting device, LED, speaker, data processing device, memory, data transmitting device, heating or cooling element, an induction loop, camera, user interface device, and/or a positioning device.

7. A module of any of previous claims, wherein the module (100) is an autonomous unit having all electric component for a certain purpose, or wherein the module comprises an interface (106) to be electrically coupled with the garment, wherein said interface comprises a snap fastener, plug and socket, connector, or at least one electrically conductive area.

8. A module of any of previous claims, wherein the module comprises an electric component and is configured to be coupled with at least one another module of any of previous claims.

9. A module of any of previous claims, wherein the module comprises conductor wires (107) for transmitting data and/or energy, and wherein said module is configured to be coupled with at least one another module (100) of claim 1-8, or an electric or data interface of the garment (101).

10. A module of any of previous claims, wherein at least one of said electric components is fixed in a removable manner.

11. A garment (101) comprising a module (100) installed with said garment, wherein the module comprises:
- an electric component (102),
- an installation zone (103) via which said module is installed with the garment,
**characterized in that**
- said electric component (102) comprises a sensor for detecting biosignals of a user during wearing said garment, and
- said installation zone (103, 108) is arranged so that the module with the electric component is also configured to be detached (108) from the garment via said installation zone by breaking the structure of said installation zone (103) physically so that at least portion of said structure of said installation zone is remained in said garment after the breaking, but all the electric components are removed.

12. A garment of claim 11, wherein the installation zone (103) is provided on an edge or side portion of the module, at a rim portion around the module, at a cover surface of the module, or the module is encapsulated between two surface layers (104, 105), and wherein the at least one surface layer is arranged as said installation zone.

13. A garment of any of claims 11-12, wherein the garment (101) is a shirt, jacket, trousers, swimming suit, band, underwear, sock, shoe, glove, hat, and/or wherein the module is a structure portion of the garment, such as a portion of sleeve, front or back portion of a shirt, leg, pocket, brand label, elastic portion of the garment, a collar portion.

14. A garment of any of claims 11-13, wherein the electric module comprises a sensor (102) for detecting heart rate, respiration rate, posture of the user, temperature, humidity, conductivity, acceleration, or wherein the electric module additionally comprises an electric energy storage, battery, capacitor, solar cell, light emitting device, LED, speaker, data processing device, memory, data transmitting device, heating or cooling element, an induction loop, camera, user interface device, and/or a positioning device.

15. A garment of any of claims 11-14, wherein the garment comprises an interface (106) to be electrically coupled with the module, and wherein said interface comprises a snap fastener, plug and socket, connector, or at least one electrically conductive area.

16. A garment of any of claims 11-15, wherein the module is a connecting structure between at least two different portions of the garment.

17. A garment of any of claims 11-16, wherein the garment comprises two layers (104, 105) configured to encapsulate said module at least partly between said layers, and wherein at least one said layer is arranged as an installation zone.

18. A method for manufacturing the module (100) or garment (101) of any of previous claims, **characterized in that** the method comprises steps of:
- providing the module (100) with an installation zone (103) via which said module is configured to be installed with a garment in a fixed manner, where said module comprises an electric component (102) comprising a sensor for detecting biosignals of a user during wearing said garment, and
- providing said installation zone (103, 108) so that the module with the electric component is also configured to be detached (108) from the garment via said installation zone by breaking the structure of said installation zone (103) physically so that at least portion of said structure of said installation zone is remained in said garment after the breaking, but all the electric components are removed.
